# FASCICULE DE BREVET EUROPEEN

(11) **EP 1 441 723 B1**
(45) Date de publication et mention de la délivrance du brevet: **02.05.2007**
(21) Numéro de dépôt: 02785535.2
(22) Date de dépôt: 09.10.2002
(51) Int. Cl.: A61K 31/4166, A61K 31/549, A61K 45/06, A61P 9/00

(54) **UTILISATION DE L'IRBESARTAN POUR LA PREVENTION OU LE TRAITEMENT DE L'HYPERTENSION PULMONAIRE**
VERWENDUNG VON IRBESARTAN ZUR VORBEUGUNG ODER BEHANDLUNG PULMONALER HYPERTONIE
USE OF IRBESARTAN FOR THE PREPARATION OF MEDICAMENTS THAT ARE USED TO PREVENT OR TREAT PULMONARY HYPERTENSION

(30) Priorité: 26.10.2001 FR 0113936
(43) Date de publication de la demande: 04.08.2004
(73) Titulaire: Sanofi-Aventis, 75013 Paris (FR)
(72) Inventeur: COSNIER-PUCHEU, Sylvie, F-34750 Villeneuve-les-Maguelone (FR); NISATO, Dino, F-34680 Saint-Georges d'Orques (FR); ROCCON, Alain, F-34270 Sainte-Croix-de-Quintillargues (FR)
(74) Mandataire: Varady, Peter
(86) Numéro de dépôt international: PCT/FR2002/003439
(87) Numéro de publication internationale: WO 2003/035062

(56) Documents cités:
- EP-A- 0 454 511
- HAVRANEK E P: "NEW CLASS OF DRUGS OFFERS BENEFITS IN HEART FAILURE" AMERICAN JOURNAL OF MANAGED CARE, AMERICAN MEDICAL PUB., OLD BRIDGE, NJ, US, vol. 7, SUPPL, no. 4, juillet 1998 (1998-07), pages S374-S376, XP008004559 ISSN: 1096-1860
- HAVRANEK E P ET AL: "DOSE-RELATED BENEFICIAL LONG-TERM HEMODYNAMIC AND CLINICAL EFFICACY OF IRBESARTAN IN HEART FAILURE" JOURNAL OF THE AMERICAN COLLEGE OF CARDIOLOGY, XX, XX, vol. 5, no. 33, avril 1999 (1999-04), pages 1174-1181, XP001077741 ISSN: 0735-1097
- DATABASE EMBASE [en ligne] ELSEVIER SCIENCE PUBLISHERS, AMSTERDAM, NL; retrieved from STN Database accession no. 1999046413 XP002204931 & ROSENSTOCK ET AL.: "The effects of irbesartan added to hydrochlorothiazide fo the treatment of hypertension in patients non-responsive to hydrochlorothiazide alone" J. LCIN. PHARMA. THER., vol. 23, no. 6, 1998, pages 433-440,
- LAURENT S ET AL: "THE ARTERIAL WALL: A NEW PHARMACOLOGICAL AND THERAPEURIC TARGET" FUNDAMENTAL & CLINICAL PHARMACOLOGY, ELSEVIER, PARIS, FR, vol. 3, no. 10, 1996, pages 243-257, XP008004566 ISSN: 0767-3981
- GILLIS J C ET AL: "IRBESARTAN A REVIEW OF ITS PHARMACODYNAMIC AND PHARMACOKINETIC PROPERTIES AND THERAPEUTIC USE IN THE MANAGEMENT OF HYPERTENSION" DRUGS, ADIS INTERNATIONAL LTD, AT, vol. 6, no. 54, décembre 1997 (1997-12), pages 885-902, XP008004554 ISSN: 0012-6667
- MARKHAM A ET AL: "IRBESARTAN AN UPDATED REVIEW OF ITS USE IN CARDIOVASCULAR DISORDER" DRUGS, ADIS INTERNATIONAL LTD, AT, vol. 5, no. 59, 2000, pages 1187-1206, XP008004567 ISSN: 0012-6667
- CASSIS L A ET AL: "ANGIOTENSIN II AND MONOCROTALINE-INDUCED PULMONARY HYPERTENSION: EFFECT OF LOSARTAN (DUP 753), A NONPEPTIDE ANGIOTENSIN TYPE 1 RECEPTOR ANTAGONIST" JOURNAL OF PHARMACOLOGY AND EXPERIMENTAL THERAPEUTICS, AMERICAN SOCIETY FOR PHARMACOLOGY AND, US, vol. 3, no. 262, septembre 1992 (1992-09), pages 1168-1172, XP008004557 ISSN: 0022-3565
- CASSIS L ET AL: "LUNG ANGIOTENSIN RECEPTOR BINDING CHARACTERISTICS DURING THE DEVELOPMENT OF MONOCROTALINE-INDUCED PULMONARY HYPERTENSION" BIOCHEMICAL PHARMACOLOGY, PERGAMON, OXFORD, GB, vol. 1, no. 54, 1 juillet 1997 (1997-07-01), pages 27-31, XP001077742 ISSN: 0006-2952
- MEANEY-MENDIOLEA E ET AL: "CLINICAL EFFECTIVENESS OF IRBESARTAN AND IRBERSARTAN PLUS HYDROCHLOROTHIAZIDE IN WOMEN WITH MILD TO MODERATE ESSENTIAL HYPERTENSION" CLINICAL DRUG INVESTIGATION, ADIS INTERNATIONAL, AUCKLAND, NZ, vol. 6, no. 19, 2000, pages 431-439, XP008004562 ISSN: 1173-2563
- CALDERONE A ET AL: "THE AT, RECEPTOR ANTAGONIST IRBESARTAN ATTENUATES CARDIAC HYPERTROPHY BUT DOES NOT SUPPRESS FIBRONECTIN EXPRESSION IN THE RAT MODEL OF MUOCARDIAL INFARCTION" CANADIAN JOURNAL OF CARDIOLOGY, PULSUS GROUP, INC, XX, vol. SUPPL. F, no. 16, 20 octobre 2000 (2000-10-20), page 152F XP008004570 ISSN: 0828-282X
- MCLEOD A A ET AL: "DRUG TREATMENT OF PRIMARY PULMONARY HYPERTENSION" DRUGS, ADIS INTERNATIONAL LTD, AT, vol. 31, no. 2, 1986, pages 177-184, XP008013160 ISSN: 0012-6667
- DATABASE MEDLINE [en ligne] US NATIONAL LIBRARY OF MEDICINE (NLM), BETHESDA, MD, US; novembre 1999 (1999-11) SCHERMULY R T ET AL: "Low-dose systemic phosphodiesterase inhibitors amplify the pulmonary vasodilatory response to inhaled prostacyclin in experimental pulmonary hypertension." Database accession no. NLM10556112 XP002235430 & AMERICAN JOURNAL OF RESPIRATORY AND CRITICAL CARE MEDICINE. UNITED STATES NOV 1999, vol. 160, no. 5 Pt 1, novembre 1999 (1999-11), pages 1500-1506, ISSN: 1073-449X
- ROBBINS I M ET AL: "ANGIOTENSIN II MEDIATES SYSTEMIC REBOUND HYPERTENSION AFTER CESSATION OF PROSTACYCLIN INFUSION IN SHEEP" JOURNAL OF APPLIED PHYSIOLOGY, AMERICAN PHYSIOLOGICAL SOCIETY, US, vol. 85, no. 2, août 1998 (1998-08), pages 731-737, XP001133785 ISSN: 8750-7587
- GIERSBERGEN VAN P L M ET AL: "A DRUG INTERACTION STUDY BETWEEN BOSENTAN AND KETOCONAZOLE AND LOSARTAN" CLINICAL PHARMACOLOGY & THERAPEUTICS, MOSBY-YEAR BOOK, ST LOUIS, MO, US, vol. 69, no. 2, février 2001 (2001-02), page P67 XP008013098 ISSN: 0009-9236
- MASSART P-E ET AL: "ANGIOTENSIN II AND ENDOTHELIN-1 RECEPTOR ANTAGONISTS HAVE CUMULATIVE HYPOTENSIVE EFFECTS IN CANINE PAGE HYPERTENSION" JOURNAL OF HYPERTENSION, CURRENT SCIENCE, PHILADELPHIA, PA, US, vol. 16, no. 6, juin 1998 (1998-06), pages 835-841, XP008013099 ISSN: 0263-6352
- CHANNICK R N ET AL: "Effects of the dual endothelin-receptor antagonist bosentan in patients with pulmonary hypertension: a randomised placebo-controlled study" LANCET, XX, XX, vol. 358, no. 9288, 6 octobre 2001 (2001-10-06), pages 1119-1123, XP004308211 ISSN: 0140-6736
- HUY HAO DAO ET AL: "AN UPDATE ON THE STATUS OF ENDOTHELIN RECEPTOR ANTAGONISTS FOR HYPERTENSION" EXPERT OPINION ON INVESTIGATIONAL DRUGS, ASHLEY PUBLICATIONS LTD., LONDON, GB, vol. 10, no. 11, novembre 2001 (2001-11), pages 1937-1946, XP008013096 ISSN: 1354-3784

## Description

La présente invention concerne une nouvelle utilisation de l'irbésartan pour la préparation de médicaments utiles pour la préparation de médicaments destinés à prévenir ou à traiter l'hypertension artérielle pulmonaire.

L'irbésartan est un antagoniste des récepteurs AT₁ de l'angiotensine II.

Ce composé et son mode de préparation sont décrits dans les brevets EP 454 511 et US 5 270 317.

L'irbésartan, seul ou en association avec un diurétique est indiqué dans le traitement de diverses affections cardiovasculaires, notamment l'hypertension et la néphropathie diabétique.

L'hypertension artérielle pulmonaire correspond à une augmentation de la pression dans le réseau artériel pulmonaire au-delà de 35 mm de Hg ; le pronostic vital de cette maladie est dramatique. Au cours de cette maladie, le calibre des vaisseaux et des artérioles pulmonaires se rétrécit et l'élévation de la pression qui en résulte retentit sur le ventricule droit; peu à peu l'insuffisance ventriculaire droite se manifeste et s'aggrave.

L'effet du losartan, antagoniste des récepteurs AT₁ de l'angiotensine II, a été testé dans cette maladie en utilisant un modèle animal dans lequel l'hypertension pulmonaire est induite par la monocrotaline. La monocrotaline (MCT) est une toxine alcaloïde qui induit des altérations vasculaires pulmonaires conduisant au développement de l'hypertension pulmonaire à l'origine d'une hypertrophie ventriculaire droite. Cette pathologie évolutive se traduit par une mortalité quasi totale des animaux en quelques semaines. Au stade terminal, on note la présence d'oedèmes pulmonaires.

Dans ce modèle, il a été trouvé par deux groupes d'auteurs différents que le losartan n'a pas d'effet :
- L. Cassis et al. : J. Pharmacol. Exp. Therap. 1992, 262(3), 1168-1172 et Biochem. Pharmacol., 1997, 54(1), 27-31,
- R. Kreutz et al. : Clin. Exp. Hypertens., 1996, 18(1), 101-111.

De façon surprenante, on a maintenant trouvé que l'irbésartan est, lui, actif sur ce modèle d'hypertension artérielle.

Ainsi la présente invention a pour objet l'utilisation de l'irbésartan pour la préparation de médicaments utiles dans la prévention ou le traitement de l'hypertension artérielle pulmonaire correspondant à une pression dans le réseau artériel pulmonaire au-delà de 35 mm Hg.

Selon la présente invention, on peut également utiliser l'irbésartan en association avec un autre principe actif, pour la préparation de médicaments utiles pour prévenir ou traiter l'hypertension artérielle pulmonaire, choisi parmi un diurétique tel que l'hydrochlorothiazide, un aquarétique, tel qu'un antagoniste des récepteurs V₂ de la vasopressine, un vasodilatateur, un anticoagulant, un inhibiteur des phosphodiesterases, la prostacycline, ou un antagoniste des récepteurs de l'endothéline tel que le bosentan.

Pour son utilisation en tant que médicament, l'irbésartan, un de ses sels pharmaceutiquement acceptable ou un de leurs solvats, seul ou en association avec un autre principe actif, doit être formulé en composition pharmaceutique.

Dans les compositions pharmaceutiques de la présente invention pour l'administration par voie orale, sublinguale, inhalée, sous-cutanée, intramusculaire, intraveineuse, transdermique, locale ou rectale, le principe actif, seul ou en association avec un autre principe actif, peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les pilules, les poudres, les granules et les solutions ou suspensions orales, les formes d'administration sublinguale et buccale, les aérosols, les formes d'administration topique, les implants, les formes d'administration transdermique, sous-cutanée, intramusculaire, intraveineuse, intranasale ou intraoculaire et les formes d'administration rectale.

Dans les compositions pharmaceutiques de la présente invention, le principe actif ou les principes actifs sont généralement formulés en unités de dosage. L'unité de dosage contient 50 à 500 mg, avantageusement de 75 à 300 mg de principe actif par unité de dosage, pour les administrations quotidiennes, une ou plusieurs fois par jour.

Pour le traitement de l'hypertension artérielle pulmonaire, selon la présente invention, on peut également choisir un traitement par inhalation ; dans ce cas les doses inhalées sont inférieures.

Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Lorsque l'on prépare une composition solide sous forme de comprimés ou de gélules, on ajoute aux principes actifs, micronisés ou non, un mélange d'excipients pharmaceutiques qui peut être composé de diluants comme par exemple le lactose, le mannitol, la cellulose microcristalline, l'amidon, le phosphate dicalcique, de liants comme par exemple la polyvinylpyrrolidone, l'hydroxypropylméthylcellulose, des agents délitants comme la polyvinylpyrrolidone réticulée, la carboxyméthylcellulose réticulée, la croscarmellose de sodium, des agents d'écoulement comme la silice, le talc, des lubrifiants comme le stéarate de magnésium, l'acide stéarique, le tribéhénate de glycérol, le stéarylfumarate de sodium.

Des agents mouillants ou tensioactifs tels que le laurylsulfate de sodium, le polysorbate 80, le poloxamer 188 peuvent être ajoutés à la formulation.

Les comprimés peuvent être réalisés par différentes techniques, compression directe, granulation sèche, granulation humide, fusion à chaud (hot-melt).

Les comprimés peuvent être nus ou dragéifiés (par du saccharose par exemple) ou enrobés avec divers polymères ou autres matières appropriés.

Les comprimés peuvent avoir une libération flash, retardée ou prolongée en réalisant des matrices polymériques ou en utilisant des polymères spécifiques au niveau du pelliculage. ,

Les gélules peuvent être molles ou dures, pelliculées ou non de manière à avoir une activité flash, prolongée ou retardée (par exemple par une forme entérique). Elles peuvent contenir non seulement une formulation solide formulée comme précédemment pour les comprimés mais aussi des liquides ou des semi-solides.

Une préparation sous forme de sirop ou d'élixir peut contenir le principe actif ou les principes actifs conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir le principe actif ou les principes actifs en mélange avec des agents de dispersion ou des agents mouillants, ou des agents de mise en suspension, comme la polyvinylpyrrolidone ou polyvidone, de même qu'avec des édulcorants ou des correcteurs du goût.

Pour une administration rectale, on recourt à des suppositoires qui sont préparés avec des liants fondant à la température rectale par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le butylèneglycol.

Ainsi, pour préparer une solution aqueuse injectable par voie intraveineuse on peut utiliser un cosolvant, par exemple un alcool tel que l'éthanol ou un glycol tel que le polyéthylèneglycol ou le propylèneglycol, et un tensioactif hydrophile tel que le polysorbate 80 ou le poloxamer 188. Pour préparer une solution huileuse injectable par voie intramusculaire, on peut solubiliser le principe actif par un triglycéride ou un ester de glycérol.

Pour l'administration locale on peut utiliser des crèmes, des pommades, des gels, des collyres, des sprays.

Pour l'administration transdermique, on peut utiliser des patches sous forme multilaminée ou à réservoir dans lequel le principe actif est en solution alcoolique.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane, des substituts des fréons ou tout autre gaz propulseur biologiquement compatible ; on peut également utiliser un système contenant le principe actif seul ou associé à un excipient, sous forme de poudre.

Le principe actif ou les principes actifs peuvent être également présentés sous forme de complexe avec une cyclodextrine, par exemple α-, β- ou γ- cyclodextrine, 2-hydroxypropyl-β-cyclodextrine ou méthyl-β-cyclodextrine.

Le principe actif ou les principes actifs peuvent être formulés également sous forme de microcapsules ou microsphères, éventuellement avec un ou plusieurs supports ou additifs.

Parmi les formes à libération prolongée utiles dans le cas de traitements chroniques, on peut utiliser des implants. Ceux-ci peuvent être préparés sous forme de suspension huileuse ou sous forme de suspension de microsphères dans un milieu isotonique.

De façon préférentielle, l'irbésartan est administré par la voie orale, en une prise unique par jour ou par inhalation en utilisant un aérosol, une ou plusieurs fois par jour.

### Protocole expérimental

Des rats mâles, Sprague-Dawley, pesant environ 300g ont reçu une injection sous-cutanée de monocrotaline (MCT) à la dose de 80 mg/kg.

Le traitement à l'irbésartan a été initié soit 21 jours, soit 14 jours après injection de monocrotaline. L'irbésartan a été incorporé à la nourriture en poudre. Les animaux témoins ont reçu l'aliment seul.

Durant toute l'étude, les animaux ont été examinés quotidiennement.

Dans une première étude, l'irbesartan est administré seul à la dose de 50 mg/kg. Dans une seconde étude, l'irbesartan est administré seul à la dose de 30 mg/kg et en association avec l'hydrochlorothiazide (HCTZ) : irbesartan : 30mg/kg et HCTZ : 10 mg/kg.

### Etude 1 :

### RESULTATS

| Traitement initié au 21^{ème} jour : | | | |
|---|---|---|---|
| **Groupes** | **Survie au 25^{ème} jour** | **Survie au 50^{ème} jour** | **Survie en fin d'étude (57^{ème} jour)** |
| **Témoins** | 100 % (18/18) | 33 % (6/18) | 17 % (3/18) |
| **Irbésartan** | 100 % (18/18) | 72% (13/18) | 61% (11/18) |
| **50 mg/kg** | - | p=0,043 | p=0,015 |

| Traitement initié au 14^{ème} jour | | | |
|---|---|---|---|
| **Groupes** | **Survie au 25^{ème} jour** | **Survie au 50^{ème} jour** | **Survie en fin d'étude (100^{ème} jour)** |
| **Témoins** | 100 % (12/12) | 33 % (4/12) | 0 % (0/12) |
| **Irbésartan** | 100 % (12/12) | 83 % (10/12) | 50 % (6/12) |
| **50 mg/kg** | - | p=0,036 | p=0,014 |

L'irbésartan, administré à la dose de 50 mg/kg/jour soit à partir du 21^{ème} jour, soit à partir ou du 14^{ème} jour post-MCT, a augmenté significativement le temps de survie des rats traités à la MCT.

Lorsque le traitement a été initié au 21^{ème} jour, on observe à l'arrêt de l'étude que 17% des animaux témoins étaient encore en vie, versus 61 % des animaux traités à l'irbésartan (p=0,0153, test de Fisher). De plus, dans le groupe traité, il apparaît une augmentation significative de la durée de survie à partir du 35^{ème} jour par rapport au groupe témoin (p=0,0160, test de log-rank).

Lorsque le traitement a été initié au 14^{ème} jour, à l'arrêt de l'étude, alors que tous les animaux témoins étaient morts, 50 % des animaux traités à l'irbésartan étaient encore en vie (p=0,014, test de Fisher). De plus, il apparaît une augmentation significative de la durée de survie globale estimée dans le groupe traité (> 93 jours) par rapport au groupe témoin (46 jours) (p=0,0001, test de log-rank).

### Etude 2 :

### RESULTATS

| Traitement initié au 14^{ème} jour | | |
|---|---|---|
| **Groupes** | **Survie au 50^{ème} jour** | **Survie en fin d'étude (85^{ème} jour)** |
| **Témoins** | 16,7 % (4/24) | 4,2 % (1/24) |
| **Irbésartan 30 mg/kg** | 47,8 % (11/23) | 0 % (0/23) |
| **HCTZ 10 mg/kg** | 25 % (6/24) | 4,2 % (1/24) |
| **Irbésartan 30 mg/kg HCTZ 10 mg/kg** | 60,9 % (14/23) | 39,1 % (9/23) |

Cette étude, réalisée à une dose plus faible d'irbesartan que la première étude, met en évidence une augmentation de la durée de survie en fin d'étude des animaux traités avec l'association irbesartan + HCTZ, comparé à irbesartan seul (p = 0,0015, test de Fisher). La durée de survie estimée médiane est de 70 jours pour les animaux traités avec l'association versus 46 jours pour les animaux traités avec l'irbésartan seul (p = 0,0033, test de log-rank).

L'ensemble de ces résultats démontre un effet bénéfique de l'irbésartan sur la mortalité consécutive à une hypertension pulmonaire induite par injection de monocrotaline chez le rat. Cet effet bénéfique est potentialisé lorsque l'irbesartan est coadministré avec un diurétique tel que l'hydrochlorothiazide.

### EXEMPLES DE COMPRIMES

| | EXEMPLE 1 | EXEMPLE 2 | EXEMPLE 3 |
|---|---|---|---|
| Irbésartan | 75.00 mg | 150.00 mg | 300.00 mg |
| Lactose, Monohydrate | 15.38 mg | 30.75 mg | 61.50 mg |
| Cellulose microcristalline | 19.50 mg | 39.00 mg | 78.00 mg |
| Amidon de maïs prégélatinisé | 22.50 mg | 45.00 mg | 90.00 mg |
| Croscarmellose de sodium | 7.50 mg | 15.00 mg | 30.00 mg |
| Poloxamer 188 | 4.50 mg | 9.00 mg | 18.00 mg |
| Silice colloïdale hydratée | 4.12 mg | 8.25 mg | 16.50 mg |
| Stéarate de magnésium | 1.50 mg | 3.00 mg | 6.00 mg |
| Eau purifiée | qs | qs | qs |
| | 150.00 mg | 300.00 mg | 600.00 mg |

| | EXEMPLE 4 | EXEMPLE 5 |
|---|---|---|
| Irbésartan | 150.00 mg | 300.00 mg |
| Hydrochlorothiazide | 12.50 mg | 12.50 mg |
| Lactose, Monohydrate | 26.65 mg | 65.80 mg |
| Cellulose microcristalline | 45.00 mg | 90.00 mg |
| Amidon de maïs prégélatinisé | 45.00 mg | 90.00 mg |
| Croscarmellose de sodium | 15.00 mg | 30.00 mg |
| Oxyde de fer rouge | 0.30 mg | 0.60 mg |
| Oxyde de fer jaune | 0.30 mg | 0.60 mg |
| Silice colloïdale hydratée | 2.25 mg | 4.50 mg |
| Stéarate de magnésium | 3.00 mg | 6.00 mg |
| Eau purifiée | qs | |
| | 300.00 mg | 600.00 mg |

| EXEMPLE 6 | |
|---|---|
| Irbesartan micronisé | 4 mg |
| Lactose | QS 20 mg |

Pour un dispositif d'inhalation de poudres composé de 7 disques de 8 doses d'un poids de 20 mg chacune.

| EXEMPLE 7 | |
|---|---|
| Irbesartan micronisé | 1 mg |
| Lactose | QS 6 mg |

Pour un dispositif d'inhalation de poudres contenant une cartouche de 12 alvéoles contenant chacune 4 mg de formulation.

| EXEMPLE 8 | |
|---|---|
| Irbesartan micronisé | 4 mg |
| Lactose 50 microns | QS 20 mg |

Pour une gélule de taille 3 terminée à 20 mg. Boites de 30 gélules. Dispositif d'inhalation de poudres.

| EXEMPLE 9 | |
|---|---|
| Irbesartan micronisé | 600 mg |
| Fréon 12 | 14 g |

Pour un flacon pressurisé avec valve doseuse contenant 150 doses.

| EXEMPLE 10 | |
|---|---|
| Irbesartan micronisé | 600 mg |
| Fréon 11 | 4,7 g |
| Fréon 12 | 9,8 g |

Pour un flacon pressurisé avec valve doseuse contenant 150 doses.

| EXEMPLE 11 | |
|---|---|
| Irbesartan micronisé | 300 mg |
| HFA (hydrofluoroalcane) 134a | 13 g |
| Trioléate de sorbitane | 30 mg |

Pour un flacon pressurisé avec valve doseuse contenant 150 doses.

| EXEMPLE 12 | |
|---|---|
| Irbesartan micronisé | 300 mg |
| Fréon 11 | 4,7 g |
| Fréon 12 | 9,8 g |
| Acide oléique | 40 mg |

Pour un flacon pressurisé avec valve doseuse contenant 150 doses.

| EXEMPLE 13 | |
|---|---|
| Irbesartan micronisé | 600 mg |
| HCTZ | 25 mg |
| Fréon 12 | 14 g |

Pour un flacon pressurisé avec valve doseuse contenant 150 doses.

| EXEMPLE 14 | |
|---|---|
| Irbesartan micronisé | 300 mg |
| HCTZ | 25 mg |
| HFA (hydrofluoroalcane) | 13 g |
| Trioléate de sorbitane | 30 mg |

Pour un flacon pressurisé avec valve doseuse contenant 150 doses.

## Revendications

1. Utilisation de l'irbésartan pour la préparation de médicaments destinés à prévenir ou traiter l'hypertension artérielle pulmonaire, ladite hypertension artérielle pulmonaire correspondant à une pression dans le réseau artériel pulmonaire au-delà de 35 mm de Hg.

2. Utilisation selon la revendication 1 dans laquelle l'irbesartan est associé à un autre principe actif choisi parmi un diurétique tel que l'hydrochlorothiazide, un aquarétique, tel qu'un antagoniste des récepteurs V₂ de la vasopressine, un vasodilatateur, un anticoagulant, un inhibiteur des phosphodiesterases, la prostacycline, ou un antagoniste des récepteurs de l'endothéline tel que le bosentan.

3. Utilisation selon la revendication 2 dans laquelle l'irbésartan est associé à l'hydrochlorothiazide.

## Claims

1. Use of irbesartan for the preparation of medicinal products that are intended to prevent or treat pulmonary arterial hypertension, the said pulmonary arterial hypertension corresponding to a pressure in the pulmonary arterial network of above 35 mmHg.

2. Use according to Claim 1, in which the irbesartan is combined with another active principle chosen from a diuretic agent such as hydrochlorothiazide, an aquaretic agent, such as a vasopressin V₂ receptor antagonist, a vasodilator, an anticoagulant, a phosphodiesterase inhibitor, prostacyclin, or an endothelin receptor antagonist such as bosentan.

3. Use according to Claim 2, in which the irbesartan is combined with hydrochlorothiazide.

## Patentansprüche

1. Verwendung von Irbesartan zur Herstellung von Medikamenten, die zur Vorbeugung oder Behandlung von pulmonal-arterieller Hypertonie bestimmt sind, wobei die pulmonal-arterielle Hypertonie einem Druck im arteriellen Lungenkreislauf von mehr als 35 mm Hg entspricht.

2. Verwendung nach Anspruch 1, wobei Irbesartan in Verbindung mit einem weiteren Wirkstoff zum Einsatz kommt, welcher aus einem Diuretikum wie etwa Hydrochlorothiazid, einem Aquaretikum wie etwa einem Antagonisten der Vasopressin-V₂-Rezeptoren, einem gefäßerweiternden Mittel, einem Gerinnungshemmer, einem Phosphodiesterasehemmer sowie aus Prostazyklin oder einem Antagonisten der Endothelinrezeptoren wie etwa Bosentan gewählt wird.

3. Verwendung nach Anspruch 2, wobei Irbesartan in Verbindung mit Hydrochlorothiazid eingesetzt wird.
